# EUROPÄISCHE PATENTSCHRIFT

(11) **EP 2 413 855 B1**
(45) Veröffentlichungstag und Bekanntmachung des Hinweises auf die Patenterteilung: **08.10.2014**
(21) Anmeldenummer: 09776497.1
(22) Anmeldetag: 01.04.2009
(51) Int. Cl.: A61F 9/008

(54) **VORRICHTUNG ZUM SCHNEIDEN EINES FLAP IN DER KORNEA EINES AUGES**
DEVICE FOR CUTTING A FLAP IN THE CORNEA OF AN EYE
DISPOSITIF DE DÉCOUPE D'UN VOLET CORNÉEN DANS L'OEIL

(43) Veröffentlichungstag der Anmeldung: 08.02.2012
(73) Patentinhaber: WaveLight GmbH, 91058 Erlangen (DE)
(72) Erfinder: DONITZKY, Christof, 90542 Eckental/Eschenau (DE)
(74) Vertreter: von Hellfeld, Axel
(86) Internationale Anmeldenummer: PCT/EP2009/002382
(87) Internationale Veröffentlichungsnummer: WO 2010/112040

(56) Entgegenhaltungen:
- WO-A-2007/123644
- US-A1- 2003 055 497
- US-A1- 2003 212 387
- US-A1- 2008 212 623

## Beschreibung

Die Erfindung betrifft eine Vorrichtung zum Schneiden eines Flap in der Kornea eines Auges.

In der ophthalmologischen Chirurgie, insbesondere dem bekannten LASIK-Verfahren, hat sich der Begriff "Flap" auch im Deutschen durchgesetzt zur Bezeichnung eines Deckelchens, welches von der Seite her in einem vorderen Bereich der Kornea geschnitten wird. Der Flap kann dann zur Seite geklappt werden, so dass in bekannter Weise mit Laserstrahlung durch Ablation die Kornea zur Behebung von Abbildungsfehlern neu geformt werden kann.

Für das Schneiden eines solchen Flaps stehen zur Zeit zum einen das sogenannte mechanische Mikrokeratom zur Verfügung, bei dem eine oszillierende Klinge den Schnitt in der Kornea erzeugt, oder zum anderen die sogenannte Femtosekunden-LASIK, bei der ein Laser eingesetzt wird mit Strahlungspulsen, die so kurz eingestellt werden, dass die Leistungsdichte der Strahlung bei Fokussierung im Inneren der Kornea dort sogenannte Fotodisruptionen bewirkt. Durch eine Steuerung dieser Femtosekundenpulse in Raum und Zeit kann dann durch eine Vielzahl solcher Fotodisruptionen ein Schnitt in der Kornea erzeugt werden. Das ist heutzutage eine weithin bekannte Technik.

Sowohl das herkömmliche mechanische Mikrokeratom als auch der beschriebene Flap-Schnitt mit Femtosekunden-Laserpulsen haben gemeinsam, dass am Rande des Flap-Schnittes ein Scharnier verbleibt, wofür sich auch im deutschen Sprachbereich der Begriff "Hinge" eingebürgert hat. Über einen solchen Scharnierbereich, in dem die Kornea durch den Schnitt nicht durchgetrennt wird, bleibt der Flap mit der Kornea verbunden, so dass er nach Durchführung der Ablation im Stroma der Kornea wieder zurückgeklappt werden kann.

Ein solcher Hinge (Scharnier) bewirkt beim Stand der Technik eine Asymmetrie des Eingriffs in die Kornea in Bezug auf zum Beispiel die optische Achse des Auges oder eine andere auf der Kornea-Oberfläche senkrecht stehende (gedachte) Achse. Der Flap-Schnitt ist nicht rotationssymmetrisch und insbesondere nicht kreisförmig in Bezug auf eine zentrale Achse des Auges.

Nach Durchführung einer LASIK-Operation spielt der intraokulare Druck des Auges beim Heilungsprozess und auch bei der Ausbildung einer Kornea-Form eine nicht zu unterschätzende Rolle. Durch den fotorefraktiven Eingriff wird die biomechanische Struktur des Auges verändert und deshalb kann auch nach der Operation in Abhängigkeit von der geänderten biomechanischen Struktur sich das Auge verformen. Der intraokulare Druck verformt die Kornea dort stärker, wo sie mehr geschwächt ist.

Die oben beschriebene asymmetrische Schnittführung zur Erzeugung des Hinge beim Stand der Technik bewirkt eine asymmetrische Gestaltung der biomechanischen Struktur der Kornea. Der intraokulare Druck kann dann nach der Operation auch eine asymmetrische Verformung der Kornea aufgrund des asymmetrischen Flap-Schnittes bewirken bis hin zu einem induzierten Zylinder oder Aberrationen höherer Ordnung. Mit anderen Worten: Beim Stand der Technik kann die asymmetrische Schnittführung bei Erzeugung des Flaps postoperativ aufgrund des intraokularen Druckes eine uner-wünschte korneale Form erzwingen.

Die US 2003/0055497 A1 beschreibt ein Verfahren zum Einführen und Platzieren eines Implantates in die Kornea eines Patienten. Der Schnitt dort ist symmetrisch in Bezug auf eine zentrale Öffnung in der Kornea.

Die US 2003/0212387 A1 beschreibt ein Verfahren zur Erzeugung eines Schnittes in zum Beispiel einer Kornea, bei der die Schnittführung symmetrisch um eine zentrale Achse des Auges erfolgt.

Die US 2008/0212623 A1 zeigt eine Anordnung, mit der ebenfalls ein symmetrischer Schnitt um eine zentrale Achse des Auges geführt wird.

Der Erfindung liegt die Aufgabe zugrunde, eine Vorrichtung zum Schneiden eines Flap in der Kornea eines Auges bereitzustellen, bei der die Gefahr unerwünschter postoperativer Verformungen der Kornea vermindert ist.

Zur Lösung dieser Aufgabe stellt die Erfindung eine Vorrichtung gemäß Anspruch 1 bereit.

Bevorzugt wird der Schnitt im Wesentlichen symmetrisch so gesetzt, dass die biomechanische Struktur der Kornea nach der Operation auch im Wesentlichen symmetrisch ist, so dass der intraokulare Druck möglichst keine unerwünschte "Ausbeulung" auf einer Seite des Auges (in Bezug auf die optische Achse) bewirkt. Die Symmetrie wird in diesem Zusammenhang bezogen auf eine Achse, die senkrecht steht auf der Oberfläche der Kornea, insbesondere, aber nicht notwendig, die optische Achse oder die Visusachse des Auges. Wird zur Gewinnung eines möglichst großen Ablationsfeldes der Flap-Schnitt etwas asymmetrisch in Bezug auf zum Beispiel eine der vorstehend genannten Achsen gesetzt (d.h. der Abstand des Hinge von der Achse etwas vergrößert, um ein möglichst großes Ablationsfeld zu erhalten), dann beziehen sich die hier angestellten Symmetriebetrachtungen auf eine solche geringfügig in Bezug auf die optische Achse oder die Visusachse verschobene gedachte Achse.

Bevorzugt wird der Flap-Schnitt einschließlich der beschriebenen Hinterschneidung unter den Hinge-Bereich in Draufsicht auf das Auge im Wesentlichen kreisförmig gestaltet.

Nachfolgend wird ein Ausführungsbeispiel der Erfindung anhand der Zeichnung näher beschrieben.

Es zeigt:
Figur 1 schematisch eine Vorrichtung zum Schneiden eines Flap in der Kornea eines Auges;
Figur 2 eine axiale Draufsicht auf die Kornea eines Auges mit Schnittführungen gemäß dem Stand der Technik;
Figur 3 einen Schnitt entlang der Linie I-II von Figur 2;
Figur 4 eine axiale Draufsicht auf die Kornea eines Auges mit einer Flap-Schnittführung gemäß der Erfindung; und
Figur 5 einen Schnitt entlang der Linie III-IV von Figur 4.

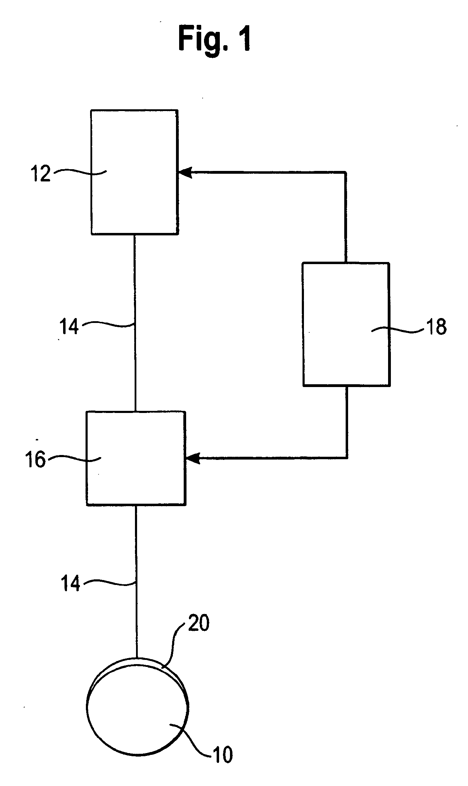

Figur 1 zeigt schematisch die wesentlichen Komponenten einer Vorrichtung zum Schneiden eines Flap in der Kornea eines Auges 10. Diese Vorrichtung ist prinzipiell gut bekannt und braucht hier deshalb nicht näher in allen Einzelheiten beschrieben zu werden. Es kann grundsätzlich auf eine bekannte Vorrichtung der Femtosekunden-LASIK (Fs-LASIK) zurückgegriffen werden und erfindungsgemäß erfolgt eine Neuprogrammierung der Computersteuerung der Fokusse der Fs-Pulse in der Kornea in neuer Weise.

Die Vorrichtung weist eine Laserstrahlungsquelle 12 auf, welche Femtosekunden-Laserstrahlungspulse 14 erzeugt. Mittel 16 dienen zur optischen Formung und Führung der Laserstrahlung 14 in Bezug auf die Kornea 20 des Auges 10. Die hierzu erforderlichen optischen Mittel und insbesondere die Mittel zur Steuerung der Strahlung in Zeit und Raum (Scanner etc.) sind gut bekannt. Eine Computersteuerung 18 steuert sowohl die Laserstrahlungsquelle 12 als auch die Mittel 16 für die Strahlungsformung und -führung.

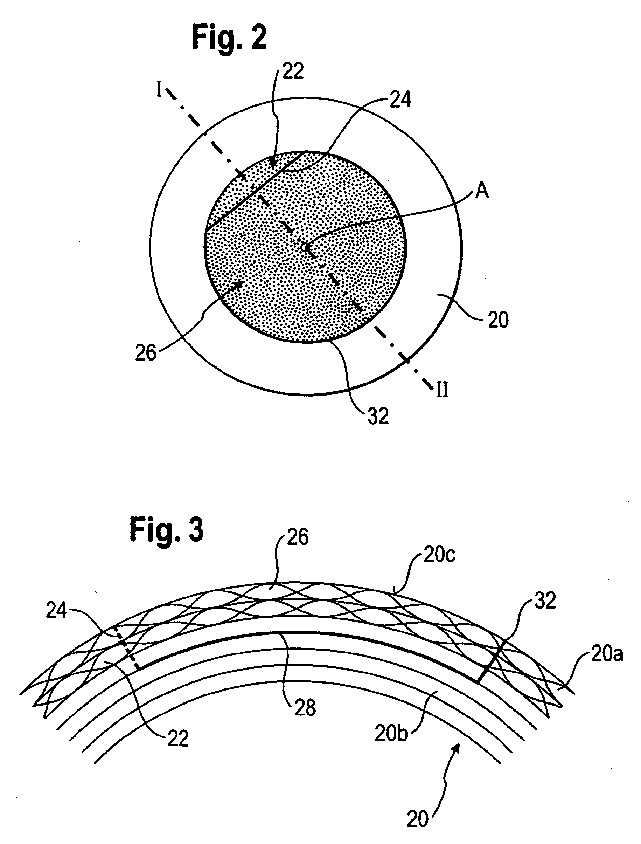

Die Figuren 2 und 3 illustrieren die Probleme beim Stand der Technik. Figur 2 zeigt eine Draufsicht in Axialrichtung auf die Kornea 20. Figur 3 zeigt den Schnitt entlang der Linie 1-2 von Figur 2.

In bekannter Weise verbleibt beim fotodisruptiv erzeugten Schnitt 28 ein Scharnierbereich 22, über den der Flap 26 mit der Kornea 20 verbunden bleibt. Die Kante des Scharnierbereichs 22 ist in den Figuren mit 24 bezeichnet. An dieser Kante 24 wird also im Stand der Technik der Flap 26 nach oben aufgeklappt. Der Schnitt 28 endet beim Stand der Technik an der Kante 24, reicht also nicht unter den Scharnierbereich 22. Auf der gegenüberliegenden Seite wird der Randschnitt 32 in bekannter Weise nach oben zur Oberfläche 20c der Kornea geführt.

Da bei den Figuren 2 und 3 der Schnitt an der Kante 24 endet, entsteht eine asymmetrische Gestaltung der biomechanischen Eigenschaften der Kornea in Bezug auf eine zentrale Achse A. Im Scharnierbereich 22 wird die Kornea weniger geschwächt als in denjenigen Bereichen, in denen der Schnitt 28 geführt ist. Figur 3 zeigt schematisch den Epithel-Bereich 20a und den Stroma-Bereich 20b der Kornea 20. Das Epithel der Kornea hat eine andere mechanische Mikrostruktur als das Stroma. Letzteres weist sogenannte Lamellen auf, die sich parallel zur Korneaoberfläche erstrecken. Diese Lamellen stellen im Wesentlichen die biomechanische Stabilität der Kornea her. Ein Schnitt durch die Lamellen stellt somit einen starken Eingriff in die biomechanische Struktur und Symmetrie des Gebildes "Auge" dar. Deshalb kann der radial wirkende intraokulare Druck nach Durchführung einer Operation mit asymmetrischer Schnittführung gemäß den Figuren 2 und 3 postoperativ eine nicht genau vorhersehbare Verformung der Kornea bewirken, insbesondere dann, wenn der ablative Eingriff relativ weit geht und das Stroma schwächt.

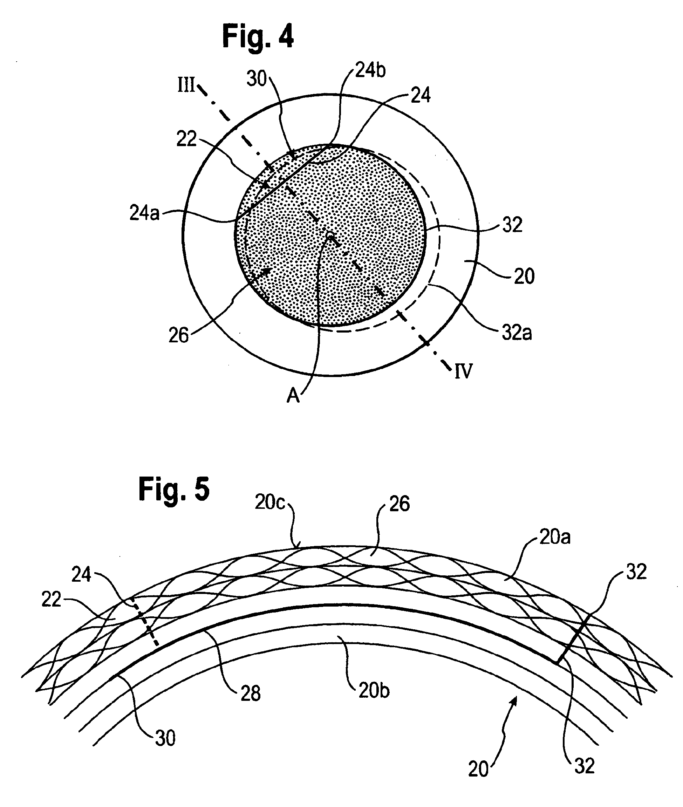

Eine solche Gefahr einer unerwünschten postoperativen Verformung der Kornea wird mit der in den Figuren 4 und 5 illustrierten Schnittführung vermieden. In den Figuren sind einander funktionsähnliche Teile und Komponenten mit den gleichen Bezugszeichen versehen. Figur 5 ist ein Schnitt entlang der Linie 3-4 von Figur 4. Gemäß den Figuren 4 und 5 endet der mit Femtosekundenpulsen fotodisruptiv erzeugte Schnitt 28 nicht an der Kante 24 des Scharnierbereichs 22, sondern wird in Form einer Hinterschneidung 30 über die Kante 24 hinaus unter den Scharnierbereich 22 geführt. An der Kante 24 erfolgt also kein Schnitt, die gepunktete Darstellung ist nur eine gedachte Linie (ebenso in Figur 3). An den beiden Endpunkten 24a, 24b bleibt somit die Verbindung zwischen Flap 26 und Kornea 30 bestehen und diese beiden Endpunkte 24a, 24b definieren somit eine Scharnier-Linie entlang der der Flap 26 aufgeklappt wird. Bei aufgeklapptem Flap 26 bleibt also die Hinterschneidung 30 unter dem unversehrt gebliebenen Scharnierbereich 22. Es wird Stroma für die anschließende ablative Bearbeitung freigelegt, ebenso wie beim oben illustrierten Stand der Technik.

Anders als beim beschriebenen Stand der Technik bewirkt aber die Hinterschneidung 30 des fotodisruptiven Schnittes einen wesentlichen symmetrischeren Eingriff in Bezug auf eine zentrale Achse A des Auges mit den oben beschriebenen Vorteilen.

Figur 4 zeigt mit dem Bezugszeichen 32a eine Abwandlung des vorstehend beschriebenen Ausführungsbeispiels. Bei dieser Abwandlung ist der Flap 26 nicht genau konzentrisch in Bezug auf das Zentrum eines Augenmerkmals, wie zum Beispiel einer Pupille. Gemäß dieser Abwandlung wird berücksichtigt, dass der Hinge-Bereich, über den der Flap mit der Kornea verbunden bleibt, eine Einschränkung des für die Ablation zur Verfügung stehenden Bereichs der Kornea bedingt. Der Scharnierbereich ist für die Laserablation nicht nutzbar. Deshalb wird gemäß dieser Variante der Erfindung der Flap-Umfang in Bezug auf die Pupille des Auges nicht konzentrisch gewählt. Zur Gewinnung einer maximalen Ablationszone wird der Umfang des Flap-Schnittes in Bezug auf das Pupillenzentrum versetzt, und zwar von der Kante 24 des Scharnierbereichs 22 weg. Dies ist in Figur 4 mit der gestrichelten Linie 32a angedeutet, die einen derart versetzten Flap-Umfang schematisch andeutet.

## Patentansprüche

1. Vorrichtung zum Schneiden eines Flap (26) in der Kornea (20) eines Auges (10), mit
- einer Laserstrahlungsquelle (12), die Laserstrahlung (14) abgibt,
- Mitteln (16, 18) einschließlich einem Rechner (18), die eingerichtet sind zum Formen und Führen der Laserstrahlung (14) in Bezug auf die Kornea (20), derart, dass
- in der Kornea ein Schnitt (32a) entsteht, der einen Scharnierbereich (22) in der Kornea (20) belässt, über den der Flap (26) mit der Kornea (20) verbunden bleibt, ein Aufklappen des Flap (26) von der Kornea (20) ermöglicht, und der sich mit einer Hinterschneidung (30) unter den Schamierbereich (22) erstreckt, **dadurch gekennzeichnet, dass**
- der Rechner (18) programmiert ist, die Fokusse der Laserstrahlung (14) und damit den Schnitt (32a) so zu führen, dass der Umfang des Schnittes in Bezug auf das Zentrum der Pupille des Auges azentrisch versetzt ist.

2. Vorrichtung nach Anspruch 1, **dadurch gekennzeichnet, dass** der Schnitt (32a) im Wesentlichen kreisförmig ist.

## Claims

1. Apparatus for cutting a flap (26) in the cornea (20) of an eye (10), comprising
- a laser radiation source (12) that is emitting laser radiation (14),
- means (16, 18) including a computer (18), adapted to form and guide the laser radiation (14) relative to the cornea (20), such that
- a cut (32a) in the cornea is generated, the cut leaving a hinge area (22) in the cornea (20) through which the flap (26) stays connected to the cornea (20) and which allows to open up the flap (26) from the cornea (20), wherein said cut extends with an undercut (30) beyond said hinge area (22),
**characterized in that**
- the computer (18) is programed such that the focuses of the laser radiation (14) and thus the cut (32a) is guided such that the circumference of the cut is offset excentrically with regard to the center of the pupil of the eye.

2. Apparatus according to claim 1, wherein said cut (32a) is essentially circular.

## Revendications

1. Dispositif de découpe d'un volet (26) dans la cornée (20) d'un oeil (10) comprenant :
- une source de rayonnement laser (12) destinée à générer un faisceau laser (14),
- des moyens (16, 18), y compris un calculateur (18), qui sont conçus pour former et guider le faisceau laser (14) par rapport à la cornée (20) de manière
- à réaliser dans la cornée une découpe (32a) qui laisse dans la cornée (20) une zone charnière (22) par le biais de laquelle le volet (26) reste relié à la cornée (20), qui permet d'ouvrir le volet (26) cornéen et qui s'étend en contre-dépouille sous ladite zone charnière (22), **caractérisé en ce que**
- le calculateur (18) est programmé pour guider les foyers du faisceau laser (14) et réaliser ainsi la découpe (32a) de telle sorte que la circonférence de cette découpe est décalée de manière acentrique par rapport au centre de la pupille.

2. Dispositif selon la revendication 1, **caractérisé en ce que** la découpe (32a) est sensiblement circulaire.
